# EUROPEAN PATENT SPECIFICATION

(11) **EP 0 729 948 B1**
(45) Date of publication and mention of the grant of the patent: **01.09.1999**
(21) Application number: 96106831.9
(22) Date of filing: 15.03.1994
(51) Int. Cl.: C07D 213/74

(54) **The production of 3-alkylamino-2-piperazinylpyridines**
Die Herstellung von 3-Alkylamino-2-Piperazinylpyridinen
La production de 3-alkylamino-2-pipérazinylpyridines

(30) Priority: 26.03.1993 US 40181
(43) Date of publication of application: 04.09.1996
(62) Divisional of application: 94912203.0
(73) Proprietor: PHARMACIA & UPJOHN COMPANY, Kalamazoo, Michigan 49001 (US)
(72) Inventor: Pearlman, Bruce Allen, Kalamazoo, Michigan 49008 (US); Krook, Mark A., Kalamazoo, Michigan 49002 (US); Perrault, William R., Kalamazoo, Michigan 49001 (US); Dobrowolski, Paul J., Kalamazoo, Michigan 49008 (US)
(74) Representative: Perry, Robert Edward

(56) References cited:
- WO-A-88/07527
- WO-A-93/01181
- BERICHTE DER DEUTSCHEN CHEMISCHEN GESELLSCHAFT, vol. 72, 1939, WEINHEIM DE, pages 933-937, XP002006871 H. RUDY AND O. MAJER: "9-Phenyl- und 9-Cyclohexyl-8-aza-flavin"
- E. KLINGSBERG: "Pyridine and its derivatives Part Three" 1962 , INTERSCIENCE PUBLISHERS , NEW YORK XP002006809 * page 60 *

## Description

### Field of the Invention

The invention relates to the preparation of 3-alkylamino-2-piperazinylpyridines.

### Background of the Invention

3-Alkylamino-2-piperazinylpyridines are useful as intermediates in the preparation of various pharmaceutical agents; see US-A-4996318, US-A-5120843 and US-A-5175281. Such agents include N-ethyl-2-[4-(5-methoxy-1H-indol-2-ylcarbonyl)-1-piperazinyl]-3-pyridineamine and 2-[4-(5-methanesulfoamido-1H-indol-2-ylcarbonyl)-1-piperazinyl]-N-(1-methylethyl)-3-pyridineamine, which are useful in treating individuals who are HIV-positive; see WO-A-9109849, Examples 16 and 105.

It is known that is is difficult to displace the chlorine of 3-amino-2-chlorosubstituted pyridines by amines. The literature teaches that it is necessary to catalyse the displacement by including copper (II) salt. Even so, yields are low, in the range of 48-59%. For example, in the presence of a catalytic amount of copper (II) sulfate, 3-amino-2-chloropyridine condenses with excess ammonium hydroxide (130°, 20 hrs) to produce 2,3-diaminopyridine in 58.9% yield, and with excess aq. methylamine (150°, 17 hrs) to produce 3-amino-2-methylaminopyridine in 48.2% yield; see Ber. 69, 2593 (1936) and DE-A-0667219. Also, in the presence of a catalytic amount of copper (II) sulfate, 3-methylamino-2-chloropyridine condenses with excess ammonium hydroxide (130°, 30 hrs) to produce 2-amino-3-methylaminopyridine in 54% yield; see J. Chem. Soc. 442(1957). With excess aq. methylamine (160°, 20 hrs), 2,3-di(methylamino)pyridine is produced in 55% yield; see J. Chem. Soc., Perkin I 673 (1973). Copper (II) sulfate also catalyses the reaction between 3-ethylamino-2-chloropyridine and piperazine (by a factor of 3-4).

Ber. 72,933 (1939), discloses the reaction of 2-chloro-3-aminopyridine with cyclohexylamine.

It is also known that strong proton acids such as hydrochloric acid catalyse the displacement of the chlorine of chloroheterocycles by aniline to form the corresponding anilino-substituted heterocycle. For example, hydrochloric acid catalyses the reaction between aniline and 2-chloro-s-triazene, see J. Am. Chem. Soc. 66, 1127 (1944); 4-chloroquinolines, see US-A-3632761, US-A-4025629 and US-A-4167567; and various 4-chloropyrimidines, see J. Chem. Soc. 1014(1949), J. Am. Chem. Soc. 66, 1127(1944) and J. Chem. Soc. 370 (1946).

However, the ability of hydrochloric acid to act as a catalyst is believed to be restricted to cases in which the displacing amine is a relatively weak base such as aniline (pKₐ = 4.6). If the pKₐ of the amine is greater than 5.1, then it has been asserted that hydrochloric acid does not catalyse the displacement, but rather simply protonates the amines; see J. Chem. Soc., 1014 (1949). For example, benzylamine (pKₐ = 9.3) does not condense with 4-chloro-(6 or 7)-nitroquinazoline in the presence of hydrochloric acid. Piperazine (pKₐ = 9.8) is an even stronger base than benzylamine.

### SUMMARY OF THE INVENTION

According to the present invention, a process for production of 3-alkylamino-2-piperazinylpyridines of formula III (see Chart A), where R₃ is -CH₂-CH₃ or CH(CH₃)₂, comprises heating a mixture of a 3-aminopyridine of formula I where X₁ is Cl or Br with piperazine, to a temperature greater than 110°C. This reaction can be conducted in the presence of a strong proton acid, and this is highly advantageous.

### DETAILED DESCRIPTION OF THE INVENTION

The present invention provides a process for producing the 3-alkylamino-2-piperazinylpyridines (III) from the corresponding 3-aminopyridine (I) and piperazine (II) as set forth in CHART A. It is preferred to obtain the 3-alkylamino-2-piperazinylpyridines (III) in water clathrate form. The 3-aminopyridines (I) and piperazine (II) are known.

For the 3-aminopyridines (I), R₃ is either ethyl -CH₂-CH₃, or isopropyl -CH(CH₃)₂. X₁ is -Cl or -Br; preferably -Cl. The 3-aminopyridine (I) is contacted with piperazine (II) and heated to greater than 110° in the absence of copper (II) ion. While the process is operable with only one equivalent of pipeline (II), it is preferable to use about 3-10, with about 5 equivalents being most preferred. It is preferred that the reaction mixture be heated to greater than 135°; more preferred that it be heated in the range of about 145 to about 170°. Since the preferred temperature range exceeds the boiling point of pipeline (II), it is preferred that the contacting and heating be conducted in a sealed system. An organic solvent can be added to the reaction mixture when the process is practiced in an open system. The solvent is added to prevent the piperazine (II) from crystallizing during reflux. When the process is practiced in a closed system, it is preferred not to have solvent present as it adds to the pressure. It is realized that a minimal amount of solvent may be present even in the closed system as a carry over from the previous reaction; in the closed system it is preferred to have none or the minimum amount of an organic solvent present. If added to the open system, it is preferred that the solvent be in the range of about 0 to about 20% by volume. Preferably the organic solvent will have a boiling point in the range of about 80 to about 150°. Suitable organic solvents include for example toluene, xylene, methanol, ethanol, isopropanol, n-propanol, n-butanol, sec-butanol and t-butanol. It is preferred that if an organic solvent is used, that it be toluene.

It is preferred to perform the process in the presence of an acid because the acid will enhance the rate of the reaction. During the course of the reaction the halogen (-X₁) and a -H (from piperazine) generate one equivalent of hydrochloric acid. Since the reaction rate is increased by acid, it is advantageous to add additional acid. It is preferred that the exogenously added acid (preferably anhydrous) be selected from the group consisting of acids with a pKₐ of 2.0 or less; it is preferred that the acid is hydrochloric or methanesulfonic. When the process is performed in the presence of an acid, it is preferred that the temperature be in the range of about 135 to about 170°. While addition of acid accelerates the rate of reaction, the desired 3-alkylamino-2-piperazinylpyridines (III) and piperazine (II) are protonated by the acid which then co-crystallize during workup and some of the desired 3-alkylamino-2-piperazinylpyridines (III) is lost. Alternatively, it is preferred to perform the process in the presence of a base (preferably anhydrous) because less product is lost during workup. Operable bases are those whose conjugate acid has a pKₐ of about 5 or greater. It is preferred that the base be selected from the group consisting of bases whose conjugate acid has a pKₐ of about 5 to about 14; it is more preferred that the base is carbonate. Non-aqueous acids/bases are preferred because water would depress the boiling point of the reaction mixture. When the reaction is complete, the reaction mixture is cooled, diluted with toluene and the excess piperazine, if any, crystallizes out leaving the desired 3-alkylamino-2-piperazinylpyridines (III) in the mother liquor. The desired 3-alkylamino-2-piperazinylpyridines (III) along with basic impurities such as 3-amino-2-piperazinylpyridine are extracted into aqueous acid. This extraction removes neutral impurities such as the bisadduct and unreacted 3-aminopyridine (I). The acidic extract is then basified by addition of aqueous base, seeded and the water clathrate of the 3-alkylamino-2-piperazinylpyridines (III) crystallizes out in analytically pure form, free of any of the above identified impurities. The desired 3-alkylamino-2-piperazinylpyridine (III) is isolated from the crystallization mixture by filtration, washed with water and dried to a workable solid. This solid may contain more or less water then the pure clathrate (about 3 to about 10 water molecules/3-alkylamino-2-piperazinylpyridine (III)), due to the presence of adsorbed water and/or adsorbed anhydrous 3-alkylamino-2-piperazinylpyridine (III).

3-Alkylamino-2-piperazinylpyridines (III) in impure form can be purified by crystallization from water or an aqueous mixture. The aqueous mixture can contain a water-miscible organic solvent or inorganic or organic salt. The salt is generated upon neutralization of the reaction mixture.

The polyhydrate forms of the 3-alkylamino-2-piperazinylpyridines (III) obtained by crystallization from an aqueous medium are true water clathrates and not simply mixtures of anhydrous crystals with water as evidenced by an X-ray crystallographic study of the water clathrates of 3-ethylamino-2-piperazinylpyridine and 3-isopropylamino-2-piperazinylpyridine.

### DEFINITIONS AND CONVENTIONS

The definitions and explanations below are for the terms as used throughout this entire document including both the specification and the claims.

### I. DEFINITIONS

All temperatures are in degrees Centigrade.

TLC refers to thin-layer chromatography.

HPLC refers to high-pressure liquid chromatography.

LOD refers to loss on drying.

CMR refers to C-13 magnetic resonance spectroscopy, chemical shifts are reported in ppm (δ) downfield from TMS.

NMR refers to nuclear (proton) magnetic resonance spectroscopy, chemical shifts are reported in ppm (δ) downfield from tetramethylsilane.

MS refers to mass spectrometry expressed as m/e or mass/charge unit. [P+H]⁺ refers to the positive ion of a parent plus a hydrogen atom. CI refers to chemical ionization.

When solvent pairs are used, the ratios of solvents used are volume/volume (v/v).

When the solubility of a solid in a solvent is used the ratio of the solid to the solvent is weight/volume (wt/v).

The term 3-alkylamino-2-piperazinylpyridines (III) includes 3-ethylamino-2-piperazinylpyridine and 3-i-propylamino-2-piperazinylpyridine.

### EXAMPLES

Without further elaboration, it is believed that one skilled in the art can, using the preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

### PREPARATION1 2-Chloro-3-ethylaminopyridine(I)

To a 500 ml flask is added in sequence 3-amino-2-chloropyridine (20.01 g, 0.1556 mol, 1.000 eq), anhydrous p-toluenesulfonic acid (0.080 g, 0.47 mmol, 0.0030 eq) and trimethylorthoacetate (23.36 g, 0.1945 mol, 1.25 eq). The slurry is agitated and an endotherm from about 22° to about 10° is observed. The mixture is warmed to about 30° with concomitant dissolution of starting material. The solution is maintained at 25-30° for 10 minutes, then vacuum is slowly applied to 70 mm mercury with simultaneous distillation of methanol cooling the mixture to 10°. The methanol is further distilled with heating to 30° at 70 mm mercury to give methyl-1-(2'-chloro-3'-pyridinyl)imino-ethyl ether, CMR (CDCl₃, ppm δ) 16.33, 53.44, 122.75, 130.03, 142.40, 142.99, 143.40, 163.64; NMR (CDCl₃, ppm δ) 1.82, 3.84, 7.16, 7.21, 8.08; MS (CI, CH₄) m/e = 185, 187 (100.%, P+1).

This material is diluted with toluene (150 ml) and the mixture cooled to -20°. Diisobutylaluminum hydride (50.282 g, 0.3536 mol, 2.272 eq.) is added dropwise with stirring maintaining -17 ±3°. The mixture is stirred 15 min. at -20°, at which point HPLC shows < 0.2% iminoether and a thin slurry has formed. Methanol (2.84 ml, 0.0701 mol, 0.451 eq) is then added slowly at -17 ±3° with concomitant distillation of hydrogen gas. To a 1 liter flask is added in sequence water (100 ml), and 20° baume hydrochloric acid (49.09 g, 0.431 mol, 2.770 eq). To this mixture is added the diisobutylaluminim hydride reaction mixture, via cannulation, with an exotherm to, and then maintenance of, 45-55° via ice bath cooling. This addition is done carefully to allow for distillation of isobutane from the quench mixture. The mixture is stirred at 45° for 15 min at which point a biphasal liquid mixture has formed. The cloudy lower aqueous phase is discarded. The organic layer is concentrated under 70 mm mercury vacuum at 70° to give the title compound, 2-chloro-3-ethylaminopyridine (containing 14 wt% toluene) by NMR and MS, CMR (CDCl₃, ppm δ) 14.46, 37.86, 117.21, 123.41, 136.11, 136.95, 140.87; NMR (CDCl₃, ppm δ)1.32, 3.18, 4.3, 6.87, 7.09, 7.69; MS (CI, CH₄): m/e = 157, 159(100.%, P+1).

### PREPARATION2 2-Chloro-3-isopropylaminopyridinemethanesulfonatesalt(I)

3-Amino-2-chloropyridine (26.9 kg), acetic acid (75.3 l) and acetone (258.2 l) are mixed and stirred at 20-25° under nitrogen. Sodium borohydride pellets (13.3 kg) are added portionwise, maintaining a temperature of between 25°-30°. At the completion of the addition, the mixture is stirred for 1 hour at 20-25°C. The reaction is quenched with water (403.5 l) and the pH adjusted to 8.0 by the addition of sodium hydroxide (50%). The aqueous solution is extracted with ethyl acetate (3 x 133 l), the organic extracts are combined, washed with sodium bicarbonate (5%, 140 l), saline (140 l) and concentrated. Azeotroping with heptane removes any residual isopropanol.

The residue is redissolved in THF (236 kg) and stirred at 20-25° under nitrogen. To this mixture is added methanesulfonic acid (13.5 l). If necessary, the mixture may be seeded to effect crystallization. The resulting slurry is cooled to -20°, filtered, washing with 0° tetrahydrofuran, and dried at 20-25° to give the title compound, mp = 110.5-112°; NMR (CDCl₃, ppm δ) 10.2, 8.0, 7.4, 3.7, 2.81, 1.25; CMR (CDCl₃, ppm δ) 140.9, 132.2, 131.3, 125.1, 124.5, 45.4, 39.3, 21.6.

### EXAMPLE1 3-Ethylamino-2-(1-piperazinyl)pyridinepentahydrate(III)

Piperazine (II, 70.10 g), sodium carbonate (22.55 g) and 2-chloro-3-ethylaminopyridine (I, PREPARATION 1) are mixed, a condenser with 1 atmosphere steam on it is added and the solid mixture warmed to =100° without agitation. The mixture is further heated to reflux at 144°. During the warmup, a large amount of piperazine sublimes into the upper part of the flask, but on reaching reflux this is washed back down. The mixture is stirred at reflux until complete (<1% 2-chloro-3-ethylaminopyridine remains) by HPLC (16 hrs) during which time the reflux temperature slowly rises to 152°. The reaction mixture is then cooled to 147° and toluene (250 ml) is added adiabatically and dropwise. The addition is completed at 74°. The slurry is further cooled to -3°. The solids are collected by vacuum filtration and washed with 0° toluene (2 x 50 ml). The combined filtrate and washes mixed with a toluene rinse. Water (150 ml) is added with stirring. The pH is adjusted from 10.9 to 5.4 with 37 wt% hydrochloric acid (25.17 g) to give a biphasal mixture; the upper phase is discarded. The aqueous phase is adjusted to pH 8.9 with 50 wt% aqueous sodium hydroxide (10.22 g). The mixture is cooled to 20° and seeded with a prior lot of 3-ethylamino-2-(1-piperazinyl)pyridine pentahydrate. The pH is further adjusted to > 12.5 with 50 wt% aqueous sodium hydroxide (15.49 g) while maintaining < 30°. The resultant slurry is cooled to 16°. The product is collected by vacuum filtration and slurry washed with 15° water (50 ml), then displacement washed with 10° water (50 ml). The solids are dried under 34.5 kPa (5 psi) nitrogen for 20 min to give the title compound, TLC eluant: 89/10/1 methylene chloride/methanol/29% aq. ammonia, R_{f} = .37; CMR (CDCl₃, ppm δ) 14.80, 38.13, 46.55, 50.56, 115.94, 119.87, 135.21, 137.57, 151.00; NMR (CDCl₃, ppm δ) 1.31, 1.79, 3.02, 3.12, 4.23, 6.81, 6.91, 7.71; MS (CI) m/e = 207 (100.%, P+1), LOD = 30.5%, yield = 80.1% (chemical) from (I).

### EXAMPLE2 3-Ethylamino-2-(1-piperazinyl)pyridinepentahydrate(III)WithoutA Base

Following the general procedure of EXAMPLE 1 and making non-critical variations but not using any base, but only piperazine, and heating at reflux (152-158°) for 14 hours, the title compound is obtained, 44.60 g, LOD 46.9%, 73.8% overall yield.

### EXAMPLE3 3-Isopropylamine-2-(1-piperazinyl)pyridine(III)

A mixture of 2-chloro-3-isopropylaminopyridine methanesulfonate salt (I, PREPARATION 2, 51.57 kg), piperazine (II, 99.9 kg) and toluene (9.7 l) are heated to 150°. After refluxing for 20-24 hours, the reaction mixture is cooled to 110° and toluene (380 l) is added while maintaining the reaction temperature at more than 105°. The reaction is further cooled to 0° and the precipitated solids are removed by filtration, washing with toluene (3 x 50 l). The filtrates are combined and water (180 l) is added. The pH of the aqueous is adjusted to 4.3-4.5 by the addition of concentrated hydrochloric acid. The layers are separated, retaining the aqueous phase, and the pH is adjusted to 12.5-13.0 by the addition of sodium hydroxide (50%). The aqueous phase is extracted with toluene (3 x 100 l), the organic extracts are combined and washed with saturated sodium chloride (50% saturated, 150 l). Concentration under reduced pressure provides the title compound, NMR (CDCl₃, ppm δ) 7.1, 6.8, 4.2, 3.5, 3.0, 1.2; CMR (CDCl₃, ppm δ) 151.0, 136.5, 134.8, 119.8, 116.1, 50.6, 46.6, 43.8 and 22.9.

### EXAMPLE4 3-Ethylamino-2-(1-piperazinyl)pyridinepentahydrate(III)

Piperazine (68.20 g, 0.7917 moles), hydrochloric acid (5.16 g, 0.1415 moles) and 2-chloro-3-ethylaminopyridine (26.65 g, containing 7.23% toluene, 24.72 g, 0.1579 moles) are mixed. The solid mixture is warmed to about 110° without agitation. The mixture is further heated to reflux at 152°. The mixture is stirred at reflux until the reaction is complete (<1% 2-chloro-3-ethylaminopyridine) by HPLC (8 hrs). The reflux temperature slowly rises to 168° through the course of the reaction. The mixture is slowly cooled to 126° at which point the piperazine hydrochloride precipitates. Toluene (150 ml) is then added adiabatically and dropwise with good agitation. The addition is completed at 75°. The slurry is further cooled to 3°. The solids are collected by vacuum filtration, washed with 0° toluene (2 x 40 ml) and discarded. The combined filtrate and washes are transferred to a 500 ml flask with a toluene rinse. Water (89 ml) is added with stirring. The pH is adjusted from 11.1 to 4.9 with 20° baume hydrochloric acid (28.41 g, 0.2494 mol. 1.58 eq) to give a liquid biphasal mixture. The upper phase is discarded. The lower phase is washed with toluene (100 ml). The aqueous phase is adjusted to pH 9.1 with aqueous sodium hydroxide (50%, 13.30 g, 0.1663 mol, 1.05 eq). The mixture is cooled to 25° and seeded with a prior lot of the title compound. The pH is further adjusted to > 12.5 with aqueous sodium hydroxide (50%, 11.52 g, 0.1440 mol, 0.91 eq), maintaining < 30°. The resultant slurry is cooled to 12°. The product is collected by vacuum filtration and the slurry washed with 10° water (89 ml), then displacement washed with 10° water (89 ml). The solids are dried under 34.5 kPa (5 psi) nitrogen for 30 minutes to give 3-ethylamino-2-(1-piperazinyl)pyridine pentahydrate (44.60 g, LOD = 34.7%).

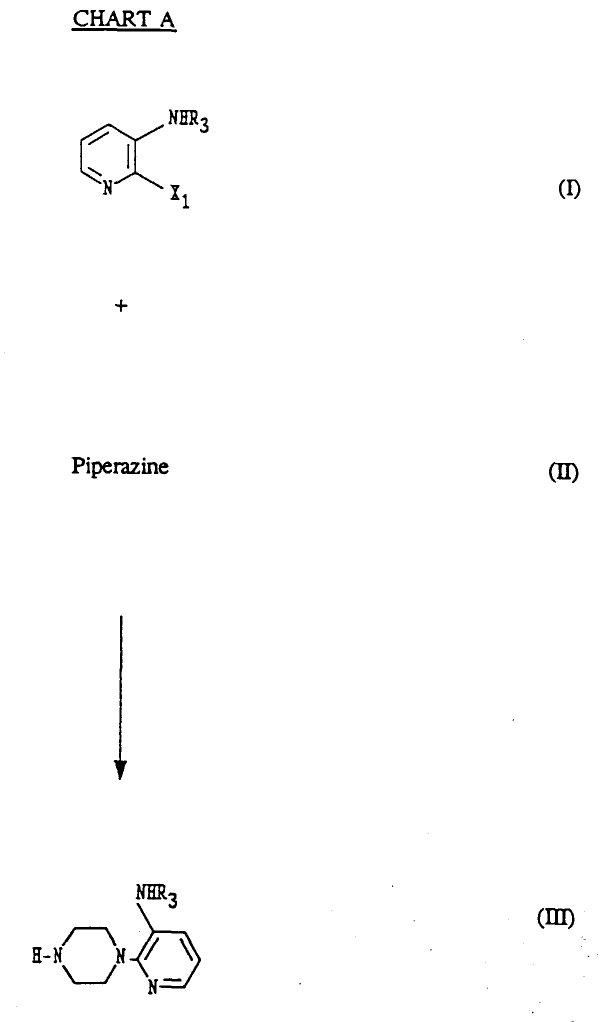

## Claims

1. A process for the production of 3-ethylamino-2-piperazinylpyridine or 3-(2-propylamino)-2-piperazinylpyridine, which comprises heating a mixture of a 3-aminopyridine of formula (I) 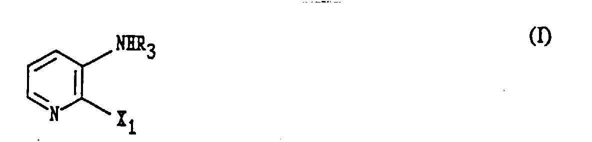 where X₁ is Cl or Br and R₃ is ethyl or 2-propyl with piperazine, to a temperature greater than 110°C.

2. A process according to claim 1, which is performed in the presence of an organic solvent selected from toluene, xylene, methanol, ethanol, n-propanol, n-butanol, sec-butanol and t-butanol.

3. A process according to claim 2, where the organic solvent is toluene.

4. A process according to any preceding claim, where the temperature is greater than 135°C.

5. A process according to claim 4, where the temperature is 145 to 170°C.

6. A process according to any preceding claim, which is performed under sealed conditions.

7. A process according to any preceding claim, which is performed in the presence of an exogenously-added acid having a pKₐ of 2.0 or less.

8. A process according to claim 7, where the acid is hydrochloric or methanesulfonic acid.

9. A process according to any of claims 1 to 6, which is performed in the presence of an exogenously-added base whose conjugate acid has a pKₐ of at least 5.

10. A process according to claim 9, where the conjugate acid has a pKₐ of 5 to 14.

11. A process according to claim 9, where the base is carbonate.

12. A process according to any preceding claim, where X₁ is Cl.

13. A process according to any preceding claim, where the product is crystallised from a mixture containing water.

## Patentansprüche

1. Verfahren zur Herstellung von 3-Ethylamino-2-piperazinylpyridin oder 3-(2-Propylamino)-2-piperazinylpyridin durch Erwärmen eines Gemischs aus einem 3-Aminopyridin der Formel (I) 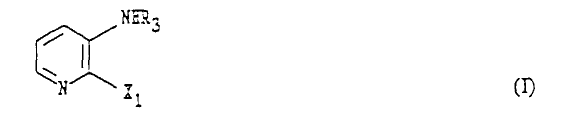 worin X₁ für Cl oder Br steht und R₃ Ethyl oder 2-Propyl darstellt, mit Piperazin auf eine Temperatur über 110°C.

2. Verfahren nach Anspruch 1, welches in Gegenwart eines organischen Lösungsmittels, ausgewählt aus Toluol, Xylol, Methanol, Ethanol, n-Propanol, n-Butanol, sek.-Butanol und tert.-Butanol, durchgeführt wird.

3. Verfahren nach Anspruch 2, wobei das organische Lösungsmittel aus Toluol besteht.

4. Verfahren nach einem der vorhergehenden Ansprüche, wobei die Temperatur über 135°C liegt.

5. Verfahren nach Anspruch 4, wobei die Temperatur 145 - 170°C beträgt.

6. Verfahren nach einem der vorhergehenden Ansprüche, welches unter abgeschlossenen Bedingungen durchgeführt wird.

7. Verfahren nach einem der vorhergehenden Ansprüche, welches in Gegenwart einer exogen zugesetzten Säure eines pKa-Werts von 2,0 oder darunter durchgeführt wird.

8. Verfahren nach Anspruch 7, wobei die Säure aus Chlorwasserstoff- oder Methansulfonsäure besteht.

9. Verfahren nach einem der Ansprüche 1 bis 6, welches in Gegenwart einer exogen zugesetzten Base, deren Konjugatsäure einen pKa-Wert von mindestens 5 aufweist, durchgeführt wird.

10. Verfahren nach Anspruch 9, wobei die Konjugatsäure einen pKa-Wert von 5 bis 14 aufweist.

11. Verfahren nach Anspruch 9, wobei die Base aus Carbonat besteht.

12. Verfahren nach einem der vorhergehenden Ansprüche, wobei X₁ für Cl steht.

13. Verfahren nach einem der vorhergehenden Ansprüche, wobei das Produkt aus einem wasserhaltigen Gemisch kristallisiert wird.

## Revendications

1. Procédé pour la production de 3-éthylamino-2-pipérazinylpyridine ou de 3-(2-propylamino)-2-pipérazinyl-pyridine, qui comprend le chauffage d'un mélange d'une 3-aminopyridine de formule (I) 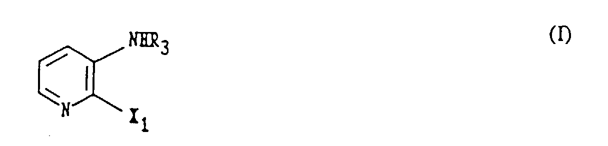 dans laquelle X₁ représente Cl ou Br et R₃ représente un groupe éthyle ou 2-propyle, avec de la pipérazine à une température supérieure à 110°C.

2. Procédé suivant la revendication 1, qui est mis en oeuvre en présence d'un solvant organique choisi entre le toluène, le xylène, le méthanol, l'éthanol, le n-propanol, le n-butanol, le sec.-butanol et le tertio-butanol.

3. Procédé suivant la revendication 2, dans lequel le solvant organique consiste en toluène.

4. Procédé suivant l'une quelconque des revendications précédentes, dans lequel la température est supérieure à 135°C.

5. Procédé suivant la revendication 4, dans lequel la température est comprise dans l'intervalle de 145 à 170°C.

6. Procédé suivant l'une quelconque des revendications précédentes, qui est mis en oeuvre dans des conditions d'étanchéité.

7. Procédé suivant l'une quelconque des revendications précédentes, qui est mis en oeuvre en présence d'un acide, ajouté de manière exogène, ayant un pKₐ égal ou inférieur à 2,0.

8. Procédé suivant la revendication 7, dans lequel l'acide consiste en acide chlorhydrique ou acide méthanesulfonique.

9. Procédé suivant l'une quelconque des revendications 1 à 6, qui est mis en oeuvre en présence d'une base ajoutée de manière exogène, dont l'acide conjugué a un pKₐ d'au moins 5.

10. Procédé suivant la revendication 9, dans lequel l'acide conjugué a un pKₐ de 5 à 14.

11. Procédé suivant la revendication 9, dans lequel la base consiste en un carbonate.

12. Procédé suivant l'une quelconque des revendications précédentes, dans lequel X₁ représente Cl.

13. Procédé suivant l'une quelconque des revendications précédentes, dans lequel le produit est cristallisé dans un mélange contenant de l'eau.
